# EUROPEAN PATENT SPECIFICATION

(11) **EP 2 266 582 B1**
(45) Date of publication and mention of the grant of the patent: **29.04.2015**
(21) Application number: 08873434.8
(22) Date of filing: 18.03.2008
(51) Int. Cl.: A61K 47/36, A61K 31/715, A61K 45/06, A61K 9/14, A61K 31/702

(54) **PHARMACEUTICAL COMPOSITION FOR PREVENTING DYSBIOSIS ASSOCIATED WITH ENTERAL ADMINISTRATION OF ANTIBIOTICS**
PHARMAZEUTISCHE ZUSAMMENSETZUNG ZUR PRÄVENTION VON DYSBIOSE IM ZUSAMMENHANG MIT ENTERALER VERABREICHUNG VON ANTIBIOTIKA
COMPOSITION PHARMACEUTIQUE ET PROCÉDÉ DE PRÉVENTION DE LA DYSBIOSE ASSOCIÉE À L'ADMINISTRATION ENTÉRALE D'ANTIBIOTIQUES

(43) Date of publication of application: 29.12.2010
(73) Proprietor: Dikovskiy, Aleksander Vladimirovich, Moscow 123182 (RU)
(72) Inventor: DOROZHKO, Oleg Valentinovich, Moscow 115446 (RU); RUDOI, Boris Anatolievich, Moscow 109518 (RU)
(74) Representative: Duncan, Garreth Andrew
(86) International application number: PCT/RU2008/000151
(87) International publication number: WO 2009/116886

(56) References cited:
- EP-A1- 1 166 800
- WO-A2-00/10582
- RU-C2- 2 277 914
- RU-C2- 2 284 832
- US-A- 5 480 491
- US-A- 5 827 526
- US-A1- 2007 254 023
- US-B2- 6 960 341
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368

## Description

### Field of industry to which the invention relates

The group of inventions relates to medicine, namely to pharmaceutics and the development of compositions of pharmaceutical preparations, containing antibiotics and prebiotics, for correcting the composition of the intestinal microflora during antibiotic therapy.

### Prior art

The therapeutic action of broad-spectrum antibiotics is as a rule accompanied by disturbances involving the gastrointestinal tract, connected with negative action of the antibiotic on the microflora of the large intestine. Antibiotics have a strong negative influence on the permeability of biological membranes to ammonium ions in the large intestine. As a result, antibiotics suppress not only pathogenic, but also useful microflora of the digestive tract, lead to disturbance of homeostasis and promote the development of dysbiosis and allergies. Disturbed equilibrium in the microbiocenosis of the intestine leads in many cases to disturbances of the immune system and active multiplication of unicellular fungi, which actively colonize the intestine. The normal intestinal microflora is a necessary condition for digestion of food and assimilation of nutrients, as well as it serves as a barrier to exogenous infection; it participates in detoxication of toxic metabolites, restriction of the multiplication of pathogenic and opportunistic microorganisms that have got into the intestine.

The most favorable conditions for the activity of microflora in the gastrointestinal-tract arise starting from the distal part of the small intestine, where the secretions of the stomach and pancreas do not reach, as well as the components of the bile, the bacteriostatic and bactericidal effects of which become weaker as the large intestine is approached. Against a background of dysbiosis, pathogenic agents of intestinal infections or opportunistic microorganisms that enter the body can quickly colonize the mucosa of the small and large intestine, destroying epithelial cells and displaying pronounced antagonism with respect to the indigenous microflora. Inflammation develops, leading to reduced production of short-chain fatty acids, which inhibit the growth of pathogenic microorganisms. This occurs during antibiotic therapy with broad-spectrum preparations. Even partial loss of the intrinsic intestinal microflora leads to serious consequences for the organism and requires specific treatment.

Such treatment can be carried out, in particular, by prescribing various probiotics, which are not always compatible with the normal microflora and over a period of several days they may be eliminated from the intestine. As for the manifestation of undesirable effects when using these preparations, they include at first and foremost the ability of probiotics to modulate immune inflammation. For example, it is known that about 10% of workers of factories producing bacterial and immunobiological preparations (probiotics), after several years of the work get allergic diseases.

A more physiological way to maintain the normal intestinal microflora in active state is intake of prebiotics. Prebiotics are indigestible components of food, which promote improvement of health by selective stimulation of the growth and/or metabolic activity of one or more groups of bacteria living in the large intestine. Carbohydrate prebiotics, in contrast to probiotics, are not digested in the stomach and are not absorbed, but reach the large intestine unchanged, because their structure has unusual beta-glycosidic bonds, which are not hydrolyzed by the human organism owing to the absence of specific beta-glycosidases. But prebiotics can selectively stimulate the growth and multiplication of lactobacilli and bifidobacteria, i.e. species that predominate in the composition of the normal microflora of the human gut. Prescribing combination therapy including prebiotics mean to eliminate atrophic processes in the mucosa of the large intestine and dystrophic changes of the epithelium with restoration of its functionality. However, in not less than in half of cases, taking antibiotics and prebiotics at different times we cannot completely preclude damage of the intestinal microflora by the antibiotics. Most often prebiotics are prescribed when the symptoms of dysbiosis have already appeared, particularly in the form of diarrhea and flatulence. As a result, by the time prebiotics begin to be taken after antibiotic therapy has been carried out, the useful microflora has been strongly disrupted or is practically unviable.

Accordingly, it is desirable to provide selective advantages for the useful microflora over pathogenic or opportunistic species of bacteria during treatment with antibiotics. Therefore there have been attempts to provide protection for the indigenous intestinal microflora by simultaneous ingestion of an antibiotic and a prebiotic in the form of a single pharmaceutical composition.

There is a known pharmaceutical composition, the method of preparation and the method of use thereof, containing the prebiotic lactulose and an antibiotic from the group comprising penicillins, cephalosporins, tetracyclines, lincosamides, macrolides (RU No. 2284832, publ. 10.10.2006, the prior art).

A drawback of this composition is that the commonly used lactulose preparations contain a considerable amount of admixtures (lactose, galactose, fructose), which can stimulate the growth of pathogenic and opportunistic species of microorganisms living parasitically in the intestine.

One more negative factor in this case is the laxative action of lactulose, which shortens the transit time of the chyme and reduces the absorption and assimilation of nutrients, and furthermore, the laxative effect of lactulose in combination with antibiotics may be falsely clinically assessed as a sign of dysbacteriosis. Moreover, it is known that some preparations of dry lactulose is extremely hygroscopic, and this creates serious technical difficulties in production of the compositions, and packaging and storage of finished pharmaceutical preparations containing lactulose.

There is a known pharmaceutical form - pharmaceutical composition, method of preparation and method of use thereof - containing an antibiotic and the fructanprebiotic, which, in addition to antibacterial action and to some, more slowly developing maintenance of the intestinal microflora, increases calcium absorption and bone mineralization (EP 1166800, 2002, prior art).

Drawbacks of this composition are a narrow range of application, low specificity of stimulant action on the main species of the indigenous microflora, nonoptimal weight ratio (lack of balance) of antibiotic and fructan, nonoptimal degree of dispersion (particle size), nonoptimal degree of polymerization of the prebiotic, at which the level of fermentation of carbohydrates and the antibacterial action of the product are reduced. There is reduced therapeutic-prophylactic effect from using the composition, as well as reduced calcium absorption and bone mineralization. Moreover, said composition is characterized by complexity of the production process and insufficient efficacy in application.

The drawbacks of this composition are largely due to the high degree of polymerization and low purity of the fructan, which hamper the process of fermentation of the polysaccharide by lactobacilli and bifidobacteria, as well as necessity to use a larger amount of prebiotic in the composition. As mentioned above negative feature is presence in lactulose preparations from the majority of domestic manufacturers of admixtures (up to 40%) that stimulate the growth of pathogenic and opportunistic bacteria of the intestinal group, the presence of side action on the blood coagulation system (mainly prolongation of the partial prothrombin time and decrease in level of fibrinogen), and high frequency of allergic reactions.

### Disclosure of the essence of the invention

The technical object of the group of inventions, bound by a common inventive concept, is the creation of an effective pharmaceutical composition and a pharmaceutical composition for use in preventing dysbiosis, while extending the arsenal of pharmaceutical compositions of preventing dysbiosis.

The technical result that enables this object to be achieved comprises expanding the range of application of a composition of prebiotics and antibiotics, by the inclusion of more effective antibacterial preparations for oral administration (fluoroquinolones, ansamycins etc.) in the composition and the elimination of side-effects. Effective utilization of the prebiotic component of the composition in the intestine is provided by administering oligosaccharides with an optimal degree of polymerization and optimal proportions of the components with the necessary degree of dispersion (particle size).

The essence of the invention with respect to a pharmaceutical composition for preventing intestinal dysbiosis during antibiotic therapy, destined for oral use, according to a first embodiment contains an antibiotic and a prebiotic, moreover the antibiotic and the prebiotic are incorporated in the form of powder, and the prebiotic comprises an oligosaccharide selected from the group comprising fructooligosaccharides, galactooligosaccharides, xylooligosaccharides, maltooligosaccharides and isomaltooligosaccharides with degree of polymerization from 2 to 10, particle size up to 0.3 mm and purity of at least 95%, and antibiotic with particle size from 20 to 200 µm, moreover the antibiotic and the oligosaccharide are incorporated in a fixed composition at a weight ratio from 1:1 to 1:100, respectively.

Preferably it contains pharmaceutically acceptable amounts of excipients, for improving the organoleptic and consumer properties, selected from the groups: fillers, taste correctants, flavorings, and odoriferous substances. The composition is produced in a pharmaceutical form suitable for oral use, selected from the group comprising capsules, tablets, powders, pills, sugar-coated pills, granules, sachets, gels, pastes, syrups, emulsions, suspensions, solutions.

The essence of the invention with respect to a pharmaceutical composition for preventing intestinal dysbiosis during antibiotic therapy, destined for oral use, according to a second embodiment contains an antibiotic and a prebiotic, said antibiotic and said prebiotic being included in the form of powder, moreover the antibiotic, selected from the group comprising beta-lactams, including combinations of beta-lactams with inhibitors of bacterial beta-lactamases, azalides, fluoroquinolones, amphenicols, glycopeptides, ansamycins, nitrofurans, derivatives of phosphonic acid, cycloserine, trimetoprim, is included with particle size from 20 to 200 µm, and oligosaccharide with degree of polymerization from 2 to 10 and with a purity of at least 95 % is included as prebiotic, moreover the antibiotic and the oligosaccharide are included in the composition at a weight ratio from 1:1 to 1:100. Preferably it contains pharmaceutically acceptable amounts of excipients, for improving the organoleptic and consumer properties, selected from the group comprising fillers, taste correctants, flavorings, and odoriferous substances. The composition is prepared in a pharmaceutical form suitable for oral use, selected from the group comprising capsules, tablets, powders, pills, sugar-coated pills, granules, sachets, gels, pastes, syrups, emulsions, suspensions, solutions.
The essence of the invention with respect to the pharmaceutical composition for use in preventing intestinal dysbiosis during antibiotic therapy according to first variant comprises administration of a fixed pharmaceutical composition containing an antibiotic and a prebiotic, said antibiotic and said prebiotic being included in the form of powder, moreover an oligosaccharide prebiotic selected from the group comprising fructooligosaccharides, galactooligosaccharides, xylooligosaccharides, maltooligosaccharides or isomaltooligosaccharides with degree of polymerization from 2 to 10, with particle size up to 0.3 mm and purity of at least 95%, and the antibiotic has a particle size from 20 to 200 µm, moreover the antibiotic and the oligosaccharide are included in a fixed composition at a weight ratio from 1:1 to 1:100, respectively, which is administered orally.

The essence of the invention with respect to the pharmaceutical composition for use in preventing intestinal dysbiosis during antibiotic therapy according to mentioned mode comprises administration of a pharmaceutical composition containing an antibiotic and a prebiotic, said antibiotic and said prebiotic being included in the form of powder, the antibiotic being selected from the group comprising beta-lactams, including combinations of beta-lactams with inhibitors of bacterial beta-lactamases, azalides, fluoroquinolones, amphenicols, glycopeptides, ansamycins, nitrofurans, derivatives of phosphonic acid, cycloserine, trimetoprim and included with particle size from 20 to 200 µm, and an oligosaccharide with degree of polymerization from 2 to 10 and with a purity of at least 95 % is included as prebiotic, moreover the antibiotic and the oligosaccharide are included in the composition at a weight ratio from 1:1 to 1:100, which is taken orally.

Use of this combination greatly increases calcium absorption and increases bone mineralization in patients. Additionally the oligosaccharides - fructooligosugars, galactooligosugars, xylooligosugars, maltooligosugars and isomaltooligosugars - not only create conditions for growth of useful bacteria, but also effectively improve the composition of the blood, and the state of the cardiovascular and immune systems.

It has to be borne in mind that the human body is a multiorgan system, the cellular elements of which are specialized for performing various functions. Interaction inside the body is based on complex regulating and coordinating mechanisms involving neurohumoral and other factors. The many separate mechanisms regulating intracellular and intercellular interactions perform opposing functions, which balance one another. This leads to the establishment of a dynamic physiological balance in the body and enables the system as a whole to maintain relative dynamic equilibrium, despite changes in the surroundings and shifts arising during the activity of the organism. Disturbance of physiological balance, including that connected with disturbance of equilibrium in microbiocenosis, may be manifested as diseases of various organs. The proposed composition and composition for use are directed to prevention or effective decrease of deviations of physiological balance, with respect to the state of the intestinal microbiocenosis under the influence of a "disturbing" factor in the form of antibiotics.

Oligosaccharides are carbohydrates whose molecules are predominantly formed by not more than 10 monosaccharide residues. They are divided into disaccharides, trisaccharides and so on. In living organisms oligosaccharides are formed during enzymatic cleavage of polysaccharides. The microorganisms in the gut utilize oligosaccharides with the aid of their own glycosidases, and oral administration of oligosaccharides leads to increased production and intensification of the saccharolytic activity of these enzymes.

Since the prebiotic - oligosaccharide in the form specified according to the present invention - is used in the proposed composition simultaneously with the antibiotic and in the necessary proportions by weight, although the antibiotic suppresses pathogenic bacteria, the intrinsic microflora of the large intestine does not perish, but synchronously with the supply of oligosaccharide, hydrolyzes (ferments) the latter with formation of an effective amount of short-chain fatty acids (predominantly lactic, partially formic and acetic). The osmotic pressure in the large intestine increases to 6.6-8.0 atm and the pH falls below 5.0, i.e. in the direction of increasing acidity, which leads to reliable maintenance of the normal selective permeability of the biological membranes of the intestine and retention of ammonium ions therein, removal of ammonia from the blood into the intestine and its ionization. Thus, in the lumen of the large intestine, conditions develop that are completely unfavorable for the development of pathogenic species of bacteria, e.g. salmonella.

The accumulated acid products and some metabolites suppress the development of a wide range of putrefactive microflora. As a result, in the lumen of the intestine there is a decrease in the quantity of pathogenic bacteria and toxic metabolites (ammonia, skatole, indole etc.). Against a background of effective maintenance of homeostasis, there is unhindered provision of sufficient multiplication and stimulation of growth of the natural useful intestinal microflora that is to be preserved. As the acidity of the medium increases, the acids react with the amino groups of protein and, by removing OH ions, promotes development of electropositive protein, which suppresses inflammatory processes that might occur in the intestine owing to external causes and as a complication of the main disease.

Any nonliving and living matter (organism, system, organ, tissue, cell, cellular organelles and substrates etc.) has its particular spectrum of electromagnetic vibrations in a wide range from hundredths of a hertz to kilo- and mega-hertz and more complex harmonics. In normal conditions, it is accepted to call these vibrations harmonic (physiological), whereas in pathologic conditions, disharmonic (pathological) vibrations appear. Oligosaccharides, in the form specified according to the present invention, being vegetable components, have energy components that initiate super-weak electromagnetic vibrations, which are superposed on the disharmonic vibrations introduced by the antibiotics, and at selected proportions by weight of the ingredients there is, as it were, "obliteration" of this potentially pathologic information.

This action of oligosaccharides is evidently also connected with an immunostimulating effect, which increases the body's nonspecific resistance to infections and preserves "biological equilibrium". There is then restoration of self-regulating processes and intensification of the harmonic vibrations that stimulate the processes of regeneration of the intestinal mucosa.

The process for preparation of the proposed composition comprises preparing specified amounts of powdered antibiotic and powdered prebiotic with a supplier-guaranteed purity of at least 95%, predrying to 2-3% moisture and mixing in the proportions specified by the present invention. The mixture also includes anticaking additives, flavorings, and taste correctants, and static electric charges are removed.

Next, packaging of the finished product is carried out, according to the dosage and the pharmaceutical form.

Compositions with the following combinations of ingredients were prepared.

Fructooligosugars with one of the amphenicols, with the oligosaccharide in the form of powder with particle size of 0.1-0.3 mm and with degree of polymerization from 2 to 6, and with the antibiotic in the form of powder with particle size of 130-200 µm, the antibiotic and the oligosaccharide being used in a weight ratio of 1:1.5.

Fructooligosugars with one of the fluoroquinolones, with the oligosaccharide in the form of powder with particle size of 0.1-0.3 mm and with degree of polymerization from 2 to 6, and with the antibiotic in the form of powder with particle size of 30-120 µm, the antibiotic and the oligosaccharide being used in a weight ratio of 1:2.

Fructooligosugars with one of the glycopeptides, with the oligosaccharide in the form of powder with particle size of 0.1-0.3 mm and with degree of polymerization from 2 to 6, and with the antibiotic in the form of powder with particle size 20-90 µm, the antibiotic and the oligosaccharide being used in a weight ratio of 1:4.

Fructooligosugars with one of the ansamycins, with the oligosaccharide in the form of powder with particle size of 0.1-0.3 mm and with degree of polymerization from 4 to 10, and with the antibiotic in the form of powder with particle size of 20-140 µm, the antibiotic and the oligosaccharide being used in a weight ratio of 1:15.

Fructooligosugars with one of the amphenicols, with the oligosaccharide in the form of powder with particle size of 0.1-0.3 mm and with degree of polymerization from 2 to 6, and with the antibiotic in the form of powder with particle size of 20-90 µm, the antibiotic and the oligosaccharide being used in a weight ratio of 1:30.

Fructooligosugars with one of the nitrofurans, with the oligosaccharide in the form of powder with particle size of 0.1-0.3 mm and with degree of polymerization from 2 to 8, and with the antibiotic in the form of powder with particle size of 20-120 µm, the antibiotic and the oligosaccharide being used in a weight ratio of 1:70.

Fructooligosugars with one of the sulfanilamide preparations (biseptol), with the oligosaccharide in the form of powder with particle size of 0.2-0.3 mm and with degree of polymerization from 2 to 6, and with the antibiotic in the form of powder with particle size of 20-120 µm, the antibiotic and the oligosaccharide being used in a weight ratio of 1:100.

Galactooligosugars with one of the amphenicols, with the oligosaccharide in the form of powder with particle size of 0.1-0.3 mm and with degree of polymerization from 5 to 15, and with the antibiotic in the form of powder with particle size of 50-150 µm, the antibiotic and the oligosaccharide being used in a weight ratio of 1:2.

Galactooligosugars with one of the fluoroquinolones, with the oligosaccharide in the form of powder with particle size of 0.1-0.3 mm and with degree of polymerization from 4 to 12, and with the antibiotic in the form of powder with particle size of 20-90 µm, the antibiotic and the oligosaccharide being used in a weight ratio of 1:3.

Galactooligosugars with one of the glycopeptides, with the oligosaccharide in the form of powder with particle size of 0.1-0.3 mm and with degree of polymerization from 5 to 15, and with the antibiotic in the form of powder with particle size of 30-100 µm, the antibiotic and the oligosaccharide being used in a weight ratio of 1:40.

Galactooligosugars with one of the ansamycins, with the oligosaccharide in the form of powder with particle size of 0.1-0.3 mm and with degree of polymerization from 3 to 10, and with the antibiotic in the form of powder with particle size of 20-110 µm, the antibiotic and the oligosaccharide being used in a weight ratio of 1:60.

Galactooligosugars with one of the derivatives of phosphonic acid (fosfomycin), with the oligosaccharide in the form of powder with particle size of 0.1-0.3 mm and with degree of polymerization from 4 to 12, and with the antibiotic in the form of powder with particle size of 20-90 µm, the antibiotic and the oligosaccharide being used in a weight ratio of 1:90.

Galactooligosugars with one of the nitrofurans, with the oligosaccharide in the form of powder with particle size of 0.1-0.3 mm and with degree of polymerization from 3 to 10, and with the antibiotic in the form of powder with particle size of 90-200 µm, the antibiotic and the oligosaccharide being used in a weight ratio of 1:55.

Galactooligosugars with one of the sulfanilamide preparations (Streptocid), with the oligosaccharide in the form of powder with particle size of 0.2-0.3 mm and with degree of polymerization from 2 to 6, and with the antibiotic in the form of powder with particle size of 40-150 µm, the antibiotic and the oligosaccharide being used in a weight ratio of 1:45.

Xylooligosugars with one of the amphenicols, with the oligosaccharide in the form of powder with particle size of 0.2-0.3 mm and with degree of polymerization from 2 to 6, and with the antibiotic in the form of powder with particle size of 20-120 µm, the antibiotic and the oligosaccharide being used in a weight ratio of 1:45.

Xylooligosugars with one of the fluoroquinolones, with the oligosaccharide in the form of powder with particle size of 0.1-0.3 mm and with degree of polymerization from 2 to 8, and with the antibiotic in the form of powder with particle size of 20-120 µm, the antibiotic and the oligosaccharide being used in a weight ratio of 1:80.

Xylooligosugars with one of the glycopeptides, with the oligosaccharide in the form of powder with particle size of 0.2-0.3 mm and with degree of polymerization from 4 to 10, and with the antibiotic in the form of powder with particle size of 160-200 µm, the antibiotic and the oligosaccharide being used in a weight ratio of 1:100.

Xylooligosugars with one of the ansamycins, with the oligosaccharide in the form of powder with particle size of 0.1-0.3 mm and with degree of polymerization from 2 to 8, and with the antibiotic in the form of powder with particle size of 20-100 µm, the antibiotic and the oligosaccharide being used in a weight ratio of 1:65.

Xylooligosugars with one of the derivatives of phosphonic acid (fosfomycin), with the oligosaccharide in the form of powder with particle size of 0.2-0.3 mm with degree of polymerization from 4 to 10, and with the antibiotic in the form of powder with particle size of 20-100 µm, the antibiotic and the oligosaccharide being used in a weight ratio of 1:5.5.

Xylooligosugars with one of the nitrofurans, with the oligosaccharide in the form of powder with particle size of 0.1-0.3 mm and with degree of polymerization from 2 to 6, and with the antibiotic in the form of powder with particle size of 30-120 µm, the antibiotic and the oligosaccharide being used in a weight ratio of 1:3.5.

Xylooligosugars with one of the sulfanilamide preparations, with the oligosaccharide in the form of powder with particle size of 0.1-0.3 mm and with degree of polymerization from 2 to 6, and with the antibiotic in the form of powder with particle size of 20-90 µm, the antibiotic and the oligosaccharide being used in a weight ratio of 1:2.

Maltooligosugars with one of the amphenicols, with the oligosaccharide in the form of powder with particle size of 0.1-0.3 mm and with degree of polymerization from 2 to 6, and with the antibiotic in the form of powder with particle size of 120-180 µm, the antibiotic and the oligosaccharide being used in a weight ratio of 1:1.

Maltooligosugars with one of the fluoroquinolones, with the oligosaccharide in the form of powder with particle size of 0.2-0.3 mm and with degree of polymerization from 2 to 6, and with the antibiotic in the form of powder with particle size of 20-120 µm, the antibiotic and the oligosaccharide being used in a weight ratio of 1:6.

Maltooligosugars with one of the glycopeptides, with the oligosaccharide in the form of powder with particle size of 0.1-0.3 mm and with degree of polymerization from 2 to 6, and with the antibiotic in the form of powder with particle size of 20-90 µm, the antibiotic and the oligosaccharide being used in a weight ratio of 1:25.

Maltooligosugars with one of the derivatives of phosphonic acid (fosfomycin), with the oligosaccharide in the form of powder with particle size of 0.1-0.3 mm and with degree of polymerization from 2 to 8, and with the antibiotic in the form of powder with particle size of 20-120 µm, the antibiotic and the oligosaccharide being used in a weight ratio of 1:60.

Maltooligosugars with one of the ansamycins, with the oligosaccharide in the form of powder with particle size of 0.1-0.3 mm and with degree of polymerization from 5 to 15, and with the antibiotic in the form of powder with particle size of 20-90 µm, the antibiotic and the oligosaccharide being used in a weight ratio of 1:70.

Maltooligosugars with one of the monobactams, with the oligosaccharide in the form of powder with particle size of 0.1-0.3 mm and with degree of polymerization from 4 to 12, and with the antibiotic in the form of powder with particle size of 40-140 µm, the antibiotic and the oligosaccharide being used in a weight ratio of 1:100.

Maltooligosugars with one of the sulfanilamide preparations, with the oligosaccharide in the form of powder with particle size of 0.1-0.3 mm and with degree of polymerization from 2 to 8, and with the antibiotic in the form of powder with particle size of 20-120 µm, the antibiotic and the oligosaccharide being used in a weight ratio of 1:6.

The composition was prepared in pharmaceutical forms suitable for oral use, in particular in the form of capsules, tablets, powders, pills, sugar-coated pills, granules, sachets, gels, pastes, syrups, emulsions, suspensions, solutions. The composition included pharmaceutically acceptable amounts of excipients for improving the organoleptic and consumer properties, in particular fillers, taste correctants, flavorings etc.

To investigate the action of the preparations obtained and to confirm their suitability to serve as prophylactic and therapeutic pharmaceutical agents, their effects on the physical state of patients with various infectious diseases were investigated. The action of the preparations on general state, physical activity, sleep, appetite, progression of atherosclerosis, neurologic status and the course of chronic somatic diseases (diabetes mellitus) was assessed.

The test group under observation comprised 157 patients in the age range from 19 to 70 years: 75 men and 82 women. The diagnosis was established in outpatient conditions based on medical examination, results of laboratory and biochemical tests, ECG data, echocardiography etc.

There were 48 patients with a diagnosis of gastric ulcer, 45 patients with a diagnosis of chronic bronchitis and bronchiectatic disease, 40 patients with a diagnosis of acute or chronic pneumonia, and 24 women with gynecological diagnoses. Many of these patients had had symptoms of gastrointestinal disturbances for a long time (colitis, enterocolitis, IBS etc.), as well as symptoms of atherosclerosis of varying severity.

Before starting intake of combination of antibiotics andprebiotics a general blood analysis and biochemical indices of the blood serum of all patients were investigated: namely AST and ALT activity, concentration of serum creatinine, glucose, calcium, total bilirubin, protein, serum iron, TIBC, sodium, potassium, cholesterol, uric acid, urea, albumin, activity of alkaline phosphatase and GGT, content of triglycerides, β-lipoproteins, and in addition urine analysis, microbiologic analysis of the intestinal contents and investigation of the feces were carried out.

The patients took the preparations 2-3 times a day, during or after a meal in amounts specified in the instructions for use of the antibiotics and the clinical standards for treatment of the particular infectious diseases. On average, antibiotics with oligosaccharides according to each of these embodiments were taken by patients in test subgroups of 5-6 patients. Monitoring tests were carried out for 2 months, every 8-10 days.

Moreover, the first control group - 64 patients of similar age and physical condition, with the same diseases - received antibiotics plus placebo (instead of oligosaccharide), and the second control group - 54 patients of similar age and physical condition, with the same diseases - received antibiotics with oligosaccharides sequentially, with an interval of 1-1.5 hours.

For the patients in both groups, during the first days of taking the preparations their state was assessed as unsatisfactory, there were observed fever, depression, chill, tinnitus, flatulence, constipation or diarrhea (the latter arose, as a rule, after previous courses of treatment with antibiotics). Improvement in general state was marked for most patients in the test group after taking the preparations for 6-8 days.

At 7^{th} - 9^{th} day after the start of treatment, the condition of the patients in the control group had also started to improve with respect to the main disease, but 75% of patients in the first group and 50% in the second group had clear symptoms of negative effects of the antibiotics on the intestinal microbiocenoses (dysbiosis), manifested as discomfort, slight pains in the region of the large intestine, flatulence and diarrhea. In some patients the dysbiosis led to reduced appetite and sleep disturbance.

By the end of the course of treatment with the preparations, there was overall improvement of condition for all the patients in the test group. There was a dramatic improvement in the state of 46 patients, and for the other patients in this group the symptoms of the main disease had practically disappeared. In 28 patients with various symptoms of atherosclerosis, headaches decreased in 22 cases, dizziness in 13, tinnitus in 16, cardiac pains in 18, and arterial blood pressure had normalized in 17 cases.

For the gynecological patients in the test group, the efficacy of the treatment was assessed before its start, during the treatment and after completion, using: biopsy of cervical mucosa, cytologic and microbiological investigation. After the first four weeks of taking the preparation, appearance of the first regions of marginal epithelization of erosions was noted, and lactobacilli and bifidobacteria appeared in the microbiocenoses of the vagina, discharges decreased considerably and painful sensations had disappeared completely. The data from morphological investigation after stopping intake of the preparation indicated almost complete replacement of columnar epithelium with squamous epithelium. Smears indicated a decrease in signs of inflammation.

In nearly all the patients in the test group, the functional state of the gastrointestinal tract had normalized, and the amount of muscle fibers, fat, fatty acids, and undigested cellulose in the stool specimen was in the normal range.

Analysis of the dynamics of a number of biochemical indices showed a significant decrease in bilirubin, β-lipoproteins, triglycerides, ALT, AST. The structural-functional changes in the plasma proteins indicated enhancement of albumin binding capacity, and increase in the activity of antibodies and proteins of the complement system. The results of biochemical investigation and cell counts of the peripheral blood also indicated positive dynamics of the process.

At the same time there was an increase in the amount of urea synthesized, indicating improvement of the processes of reamination and transamination of amino acids in the liver, i.e. normalization of metabolic detoxication processes.

Nearly all patients in the test group noticed improvement in quality of life through increase in physical activity, improvement of mood and sleep, normalization of appetite and of the working of the intestines. No adverse side-effects from taking the preparation appeared during treatment or follow-up.

In the first control group, among patients who received the antibiotic plus placebo, despite the decrease in severity of the symptoms of the main disease (under the influence of the antibiotic), 88% did have no clear tendency toward improvement in the indices of intraintestinal homeostasis. In the second control group, patients who received the antibiotic and oligosaccharides sequentially, the indices of intraintestinal homeostasis were similar to the indices in the test group in 55% of cases.

Observation of the state of the majority of patients in the test group, who received the proposed preparation, was continued over the next 4-9 months and confirmed the overall decrease in incidence of respiratory diseases, decrease in level of anxiety, increase in physical capacity for work, normalization of sleep, decrease in frequency of relapses of the underlying disease and steady improvement in quality of life, especially in the bowel function.

The results presented above confirm the efficacy of prophylaxis and treatment of dysbiosis during antibacterial therapy.

As a result, variants of an effective pharmaceutical composition and variants of an effective method of preventing dysbiosis have been created, and the arsenal of pharmaceutical compositions and methods of preventing dysbiosis has been expanded.

Moreover, the range of application of a composition of prebiotics and antibiotics has been expanded by including the most effective antibacterial preparations for oral administration (fluoroquinolones and ansamycins) in the composition and by eliminating side-effects. Effective utilization of the prebiotic component of the composition in the intestine was provided by introducing oligosaccharides with an optimal degree of polymerization and optimal proportions of the components with the necessary degree of dispersion (particle size).

### Suitability for industrial application

The present invention is implemented using universal equipment, which is widely used in industry.

## Claims

1. A pharmaceutical composition for preventing intestinal dysbiosis during antibiotic therapy, destined for oral use, containing an antibiotic and a prebiotic, **characterized in that** the antibiotic and the prebiotic are included in the form of powder, and it includes, as prebiotic, an oligosaccharide selected from the group comprising fructooligosaccharides, galactooligosaccharides, xylooligosaccharides, maltooligosaccharides and isomaltooligosaccharides with degree of polymerization from 2 to 10, with particle size up to 0.3 mm and purity of at least 95%, and an antibiotic with particle size from 20 to 200 µm, the antibiotic and the oligosaccharide being included in a fixed composition at a weight ratio from 1:1 to 1:100, respectively.

2. The pharmaceutical composition as claimed in claim 1, **characterized in that** it contains pharmaceutically acceptable amount of excipients for improving the organoleptic and consumer properties, selected from the group comprising fillers, taste correctants, flavorings, and odoriferous substances.

3. The pharmaceutical composition as claimed in either of claims 1 and 2, **characterized in that** it is made in a pharmaceutical form suitable for oral use, selected from the group comprising capsules, tablets, powders, pills, sugar-coated pills, granules, sachets, gels, pastes, syrups, emulsions, suspensions, solutions.

4. A pharmaceutical composition for preventing intestinal dysbiosis during antibiotic therapy, destined for oral use, containing an antibiotic and a prebiotic, **characterized in that** the antibiotic and the prebiotic are included in the form of powder, moreover the antibiotic, selected from the group comprising beta-lactams, including combination beta-lactams with inhibitors of bacterial beta-lactamases, azalides, fluoroquinolones, amphenicols, glycopeptides, ansamycins, nitrofurans, derivatives of phosphonic acid, cycloserine, trimetoprim, is included with particle size from 20 to 200 µm, and it includes, as prebiotic, an oligosaccharide with degree of polymerization from 2 to 10 and with a purity of at least 95 %, the antibiotic and the oligosaccharide being included in the composition at a weight ratio from 1:1 to 1:100.

5. The pharmaceutical composition as claimed in claim 4, **characterized in that** it contains pharmaceutically acceptable amounts of excipients for improving the organoleptic and consumer properties, selected from the group comprising fillers, taste correctants, flavorings, and odoriferous substances.

6. The pharmaceutical composition as claimed in either of claims 4 and 5, **characterized in that** it is made in a pharmaceutical form suitable for oral use, selected from the group comprising capsules, tablets, powders, pills, sugar-coated pills, granules, sachets, gels, pastes, syrups, emulsions, suspensions, solutions.

7. A pharmaceutical composition as claimed in claim 1 for use in preventing intestinal dysbiosis during antibiotic therapy by oral administration.

8. A pharmaceutical composition as claimed in claim 4 for use in preventing intestinal dysbiosis during antibiotic therapy by oral administration.

## Patentansprüche

1. Pharmazeutische Zusammensetzung zur Verhinderung von Darmdysbiose während einer Antibiotikatherapie, bestimmt für die orale Verwendung, enthaltend ein Antibiotikum und ein Präbiotikum, **dadurch gekennzeichnet, dass** das Antibiotikum und das Präbiotikum in Pulverform eingeschlossen sind und sie als Präbiotikum ein aus der aus Fructooligosacchariden, Galaktooligosacchariden, Xylooligosacchariden, Maltooligosacchariden und Isomaltooligosacchariden bestehenden Gruppe ausgewähltes Oligosaccharid mit einem Polymerisationsgrad von 2 bis 10, mit einer Partikelgröße von bis zu 0,3 mm und einer Reinheit von mindestens 95% und ein Antibiotikum mit einer Partikelgröße von 20 bis 200 µm umfasst, wobei das Antibiotikum und das Oligosaccharid in einer fixen Zusammensetzung in einem Gewichtsverhältnis von 1:1 bis 1:100 enthalten sind.

2. Pharmazeutische Zusammensetzung nach Anspruch 1, **dadurch gekennzeichnet, dass** sie eine pharmazeutisch unbedenkliche Menge an Exzipienten ausgewählt aus der aus Füllstoffen, geschmackskorrigierenden Stoffen, Geschmacksstoffen und Geruchsstoffen umfassenden Gruppe zur Verbesserung der organoleptischen und Einnahmeeigenschaften enthält.

3. Pharmazeutische Zusammensetzung nach Anspruch 1 oder 2, **dadurch gekennzeichnet, dass** sie in einer für die orale Anwendung geeigneten pharmazeutischen Form ausgewählt aus der aus Kapseln, Tabletten, Pulvern, Pillen, Dragees, Granulaten, Beuteln, Gelen, Pasten, Sirupen, Emulsionen, Suspensionen und Lösungen bestehenden Gruppe hergestellt ist.

4. Pharmazeutische Zusammensetzung zur Verhinderung von Darmdysbiose während einer Antibiotikatherapie, bestimmt für die orale Verwendung, enthaltend ein Antibiotikum und ein Präbiotikum, **dadurch gekennzeichnet, dass** das Antibiotikum und das Präbiotikum in Pulverform eingeschlossen sind, außerdem das aus der beta-Lactame einschließlich Kombinationen von beta-Lactamen mit Inhibitoren bakterieller beta-Lactamasen, Azaliden, Fluorchinolonen, Amphenicolen, Glykopeptiden, Ansamycinen, Nitrofuranen, Derivaten von Phosphonsäure, Cycloserin und Trimetoprim umfassenden Gruppe ausgewählte Antibiotikum mit einer Partikelgröße von 20 bis 200 µm eingeschlossen ist und sie als Präbiotikum ein Oligosaccharid mit einem Polymerisationsgrad von 2 bis 10 und mit einer Reinheit von mindestens 95% einschließt, wobei das Antibiotikum und das Oligosaccharid in der Zusammensetzung in einem Gewichtsverhältnis von 1:1 bis 1:100 enthalten sind.

5. Pharmazeutische Zusammensetzung nach Anspruch 4, **dadurch gekennzeichnet, dass** sie eine pharmazeutisch unbedenkliche Menge an Exzipienten ausgewählt aus der aus Füllstoffen, geschmackskorrigierenden Stoffen, Geschmacksstoffen und Geruchsstoffen umfassenden Gruppe zur Verbesserung der organoleptischen und Einnahmeeigenschaften enthält.

6. Pharmazeutische Zusammensetzung nach Anspruch 4 oder 5, **dadurch gekennzeichnet, dass** sie in einer für die orale Anwendung geeigneten pharmazeutischen Form ausgewählt aus der aus Kapseln, Tabletten, Pulvern, Pillen, Dragees, Granulaten, Beuteln, Gelen, Pasten, Sirupen, Emulsionen, Suspensionen und Lösungen bestehenden Gruppe hergestellt ist.

7. Pharmazeutische Zusammensetzung nach Anspruch 1 zur Verwendung bei der Verhinderung von Darmdysbiose während einer Antibiotikatherapie durch orale Verabreichung.

8. Pharmazeutische Zusammensetzung nach Anspruch 4 zur Verwendung bei der Verhinderung von Darmdysbiose während einer Antibiotikatherapie durch orale Verabreichung.

## Revendications

1. Composition pharmaceutique pour prévenir la dysbiose intestinale pendant une thérapie antibiotique, destinée à une utilisation orale, contenant un antibiotique et un prébiotique, **caractérisée en ce que** l'antibiotique et le prébiotique sont inclus sous la forme de poudre, et qui comprend, en tant que prébiotique, un oligosaccharide choisi dans le groupe comprenant des fructo-oligosaccharides, des galactooligosaccharides, des xylo-oligosaccharides, des maltooligosaccharides et des isomalto-oligosaccharides avec un degré de polymérisation de 2 à 10, avec une taille de particules allant jusqu'à 0,3 mm et une pureté d'au moins 95 %, et un antibiotique ayant une taille de particules de 20 à 200 µm, l'antibiotique et l'oligosaccharide étant inclus dans une composition fixe à un rapport en poids de 1:1 à 1:100, respectivement.

2. Composition pharmaceutique selon la revendication 1, **caractérisée en ce qu'**elle contient une quantité pharmaceutiquement acceptable d'excipients pour améliorer les propriétés organoleptiques et liées au consommateur, choisis dans le groupe comprenant des charges, des correcteurs de goût, des arômes, et des substances odoriférantes.

3. Composition pharmaceutique selon l'une des revendications 1 et 2, **caractérisée en ce qu'**elle est préparée sous une forme pharmaceutique adaptée pour utilisation orale, choisie dans le groupe comprenant des capsules, des comprimés, des poudres, des pilules, des pilules dragéifiées, des granules, des sachets, des gels, des pâtes, des sirops, des émulsions, des suspensions, des solutions.

4. Composition pharmaceutique pour prévenir la dysbiose intestinale pendant une thérapie antibiotiques, destinée à une utilisation orale, contenant un antibiotique et un prébiotique, **caractérisée en ce que** l'antibiotique et le prébiotique sont inclus sous la forme d'une poudre, de plus, l'antibiotique, choisi dans le groupe comprenant des bêta-lactames, comprenant des bêta-lactames en combinaison avec des inhibiteurs de bêta-lactamases bactériennes, des azalides, des fluoroquinolones, des amphénicols, des glycopeptides, des ansamycines, des nitrofuranes, des dérivés d'acide phosphonique, la cyclosérine, le trimétroprim, est inclus avec une taille de particule de 20 à 200 µm, et elle comprend, en tant que prébiotique, un oligosaccharide avec un degré de polymérisation de 2 à 10 et avec une pureté d'au moins 95 %, l'antibiotique et l'oligosaccharide étant inclus dans la composition à un rapport en poids de 1:1 à 1:100.

5. Composition pharmaceutique selon la revendication 4, **caractérisée en ce qu'**elle contient des quantités pharmaceutiquement acceptables d'excipients pour améliorer les propriétés organoleptiques et liées au consommateur, choisis dans le groupe comprenant des charges, des correcteurs de goût, des arômes, et des substances odoriférantes.

6. Composition pharmaceutique selon l'une des revendications 4 et 5, **caractérisée en ce qu'**elle est fabriquée sous une forme pharmaceutique adaptée pour utilisation orale, choisie dans le groupe comprenant des capsules, des comprimés, des poudres, des pilules, des pilules dragéifiées, des granules, des sachets, des gels, des pâtes, des sirops, des émulsions, des suspensions, des solutions.

7. Composition pharmaceutique selon la revendication 1 pour utilisation dans la prévention de la dysbiose intestinale pendant une thérapie antibiotique par administration orale.

8. Composition pharmaceutique selon la revendication 4 pour utilisation dans la prévention de la dysbiose intestinale pendant une thérapie antibiotique par administration orale.
